# FASCICULE DE BREVET EUROPEEN

(11) **EP 1 468 321 B1**
(45) Date de publication et mention de la délivrance du brevet: **15.03.2006**
(21) Numéro de dépôt: 02801158.3
(22) Date de dépôt: 20.12.2002
(51) Int. Cl.: G02B 23/24, A61B 1/07

(54) **TETE OPTIQUE DE FOCALISATION MINIATURISEE NOTAMMENT POUR ENDOSCOPE**
MINIATURISIERTER FOKUSSIERENDER OPTISCHER KOPF INSBESONDERE FÜR EIN ENDOSKOP
MINIATURIZED FOCUSING OPTICAL HEAD IN PARTICULAR FOR ENDOSCOPE

(30) Priorité: 28.12.2001 FR 0116979
(43) Date de publication de la demande: 20.10.2004
(73) Titulaire: Mauna Kea Technologies, 75010 Paris (FR)
(72) Inventeur: BERIER, Frédéric, F-92400 Courbevoie (FR); BOURRIAUX, Stéphane, F-77420 Champs sur Marne (FR); GENET, Magalie, F-78280 Guyancourt (FR); VIELLEROBE, Bertrand, F-94130 Nogent Sur Marne (FR); LOISEAU, Alexandre, F-75007 Paris (FR); ABRAT, Benjamin, F-75008 Paris (FR)
(74) Mandataire: Pontet, Bernard
(86) Numéro de dépôt international: PCT/FR2002/004482
(87) Numéro de publication internationale: WO 2003/056379

(56) Documents cités:
- EP-A- 0 821 907
- EP-A- 0 887 043
- US-A- 5 191 879
- US-A- 5 961 445

## Description

La présente invention concerne une tête optique miniaturisée prévue pour équiper une extrémité distale d'un faisceau de fibres optiques souples, ladite tête étant destinée à être placée au contact d'une surface d'analyse et adaptée à focaliser un signal d'excitation véhiculé par ledit faisceau de fibres en un point focal d'excitation pouvant être situé à différentes profondeurs par rapport à la surface de contact de la tête. La tête optique est également adaptée à prélever un signal rétroémis provenant du point focal d'excitation subsurfacique pour qu'il soit acheminé par le faisceau de fibres notamment vers des moyens de détection et des moyens d'analyse et de traitements numériques de signal.

Les domaines d'application concernés sont les dispositifs d'analyse subsurfacique à caractère confocal, les signaux véhiculés pouvant être notamment du domaine de l'imagerie et/ou de la spectroscopie suivant la ou les sources d'excitation et les moyens de détection mis en oeuvre. Le caractère confocal résulte de l'utilisation de la ou des mêmes fibres pour véhiculer le signal d'excitation et le signal rétroémis. Il peut s'agir d'analyses biologiques in situ, sur l'homme ou l'animal, externes par exemple du domaine de la dermatologie, ou internes et accessibles à l'aide d'un canal opérateur d'endoscope dans lequel on peut introduire le faisceau de fibres optiques et la tête optique. Il peut s'agir également d'analyses cellulaires réalisées ex vivo sur des prélèvements. En outre encore, la tête optique peut être mise en oeuvre pour l'analyse de l'intérieur d'un dispositif manufacturé.

Actuellement sont visés les domaines médicaux de la gastro-entérologie, la pneumologie, la gynécologie, l'urologie, l'ORL, la dermatologie, l'ophtalmologie, la cardiologie et de la neurologie.

Le grandissement de la tête optique selon la présente invention peut être unitaire ou non. Ce sont les moyens d'analyse et de traitement de signal prévus du côté de l'extrémité proximale du faisceau de fibres optiques qui permettent de restituer une image ou un graphe interprétable par un utilisateur.

Les objectifs recherchés pour la tête optique sont notamment les suivants :
- présenter un encombrement minimal pour notamment pouvoir être inséré dans le canal opérateur d'un endoscope qui en général possède un diamètre compris entre 2 et 4 mm et un rayon de courbure donné.
- fournir un signal rétroémis de bonne qualité dans lesquels les aberrations sont minimisées ;
- minimiser les réflexions parasites en sortie distale du faisceau de fibres ;
- fournir une résolution spatiale du point focal d'excitation de l'ordre de 4µm, voir inférieure dans le cas d'un grandissement non unitaire, permettant l'analyse et/ou l'observation d'un tissu à une échelle cellulaire ;
- pouvoir être amenée au contact de la surface d'analyse afin d'éviter les problèmes liés aux déplacements intempestifs ; et
- permettre un point focalisé dans un plan de coupe XY situé à une profondeur donnée de la surface d'analyse.

Une miniaturisation de la tête optique est également avantageuse pour augmenter la précision de son positionnement et également pour minimiser l'inertie mécanique dans des applications automatisées, par exemple en extension de bras de robot ou de télémanipulateur.

On connaît par le document WO 00/16151, un appareil d'observation comprenant une tête optique de focalisation à l'extrémité distale d'un canal flexible de fibres optiques comprenant en sortie du canal successivement trois lentilles : un objectif, de microscope x 10, un doublet de focale 150mm et un doublet de focale 50mm.

On connaît également une tête optique comprenant un système de quatre lentilles, la première lentille et la quatrième lentille étant deux objectifs de microscope x10 et la deuxième et la troisième lentille deux doublets de focale 150mm constituant un système afocal de grandissement 1.

Ces systèmes optiques ont pour inconvénients majeurs :
- ce type de construction à base d'objectifs de microscopes sophistiqués (pouvant contenir jusqu'à douze lentilles) ne peut être miniaturisé pour être introduit dans un canal opérateur d'endoscope d'un diamètre de 2 à 4 mm ;
- la résolution latérale est de l'ordre de 8µm, insuffisante pour analyser un tissu à l'échelle cellulaire ;
- dans le cas d'une imagerie confocale, avec une illumination et un balayage des fibres une à une, on observe une distorsion de l'image formée ("ballonnement" des lignes).

La présente invention a pour but de pallier ces inconvénients et d'atteindre les objectifs mentionnés plus haut.

Ce but est atteint avec une tête optique miniaturisée prévue pour équiper l'extrémité distale d'un faisceau de fibres optiques souples, ladite tête optique étant destinée à venir au contact d'une surface d'analyse et comprenant des moyens optiques pour focaliser un signal d'excitation sortant dudit faisceau de fibres en un point focal dit d'excitation situé à une profondeur donnée sous la surface d'analyse et pour prélever un signal rétroémis par le point focal d'excitation qui est véhiculé en retour par ledit faisceau de fibres, caractérisée par un tube porte-optique de section circulaire au sein duquel sont introduits d'un côté la partie terminale distale du faisceau de fibres et de l'autre les moyens optiques, ces derniers comprenant une lame placée au contact de l'extrémité du faisceau de fibres dont l'indice est proche de celui du coeur des fibres et un bloc optique de focalisation, un hublot de sortie étant en outre destiné à venir au contact de la surface d'analyse et adapté à réalisé une adaptation d'indice de manière à s'affranchir de la réflexion parasite s'opérant sur la surface d'analyse.

Le bloc optique comprend un ensemble de lentilles pouvant être standards, le positionnement et la courbure de chaque lentille ne permettant pas un couplage du signal réfléchi par les lentilles, en particulier un couplage de plus de 10⁻⁵ par rapport au signal en sortie de fibre. Cela permet d'éviter que le signal provenant de l'échantillon observé ne soit parasité par ce signal réfléchi. A cet effet, chaque lentille constituant le bloc optique possède un traitement anti-reflet optimal à la longueur d'onde de travail, et, par ailleurs, il est placé dans un plan extra-focal et présente une courbure qui permettent de rejeter te signal réfléchi en dehors de la fibre d'excitation. L'association des diverses lentilles permet d'éclairer le site d'analyse au besoin point à point tout en assurant une bonne qualité optique nécessaire à l'obtention d'une image confocale hautement résolue.

Selon une première forme de réalisation, le hublot est inséré également à l'extrémité du tube porte-optique.

Selon une seconde forme de réalisation, permettant une analyse à différentes profondeurs, notamment entre 50 et 400 µm, le hublot est porté par un capuchon mobile enfilé à l'extrémité de la tête optique et déplaçable à l'aide de moyens appropriés, hydrauliques, pneumatiques, piézo-électriques, motorisés, électro-optiques, etc. dont l'encombrement reste compatible avec l'objectif de miniaturisation.

D'autres modes de déplacement de la profondeur du plan d'analyse peuvent être envisagés, notamment le déplacement axial d'un moyen optique mobile prévu dans le bloc optique, ce moyen optique mobile pouvant être constitué d'une optique réfractive (standard ou à gradient d'indice) ou d'une optique diffractive. Un moteur piézoélectrique peut réaliser le déplacement de ce moyen optique mobile. On peut également utiliser un actionneur hydraulique. Un autre mode de balayage axial peut consister également à utiliser un moyen optique spécifique du bloc optique adapté à changer la distance focale par la modification de son rayon de courbure (ou puissance optique). Ce moyen optique peut être par exemple un moyen optique liquide.

Pour l'observation et l'analyse de tissus biologiques très diffusants et/ou présentant des détails cellulaires nécessitant une très haute résolution spatiale, tels que les noyaux de cellules saines, on préfèrera une tête optique à grandissement non unitaire, notamment de 0,5 depuis l'extrémité distale du guide d'image jusqu'au plan d'analyse. Cela permet d'améliorer la résolution latérale, axiale et d'obtenir une ouverture numérique plus grande.

Ainsi, selon l'invention, pour obtenir une miniaturisation, on remplace les objectifs de microscopes choisis classiquement dans les têtes de focalisation pour leur excellente qualité optique, par une combinaison de moyens mécaniques et optiques optimisés de manière à obtenir un couplage optimal du signal en sortie de fibre, c'est à dire avec une transcription optimisée de la fonction d'étalement de point (PSF), une qualité du front d'onde limité par la diffraction (de préférence de l'ordre de λ/30 au centre du champ à λ/20 en bord de champ) pour obtenir ainsi une minimisation des aberrations dues à l'utilisation de lentilles plus standards pour le bloc optique de focalisation.

Pour l'observation de tissus peu diffusants et présentant des détails supérieurs à 5 µm, notamment pour des tissus biologiques peu diffusants ou des objets manufacturés tels que les circuits intégrés, une tête à grandissement unitaire présente l'avantage d'être plus simple à réaliser et à intégrer, grâce à son caractère symétrique, et par conséquent présente un coût inférieur à celui de la tête à grandissement non unitaire.

D'autres avantages et caractéristiques de l'invention apparaîtront à la lumière de la description qui va suivre d'un exemple de réalisation non limitatif, description faite en référence au dessin annexé sur lequel :
- la figure 1 est une vue en coupe longitudinale opto-mécanique d'une tête optique selon l'invention ;
- la figure 2 est un schéma optique illustrant un exemple de réalisation du bloc optique de focalisation à grandissement unitaire ;
- la figure 3 est un schéma optique illustrant un exemple de réalisation du bloc optique de focalisation à grandissement non unitaire ; et
- la figure 4 est une vue en coupe similaire à la figure 1 illustrant un mode de réalisation de tête optique à profondeur de champ régtable.

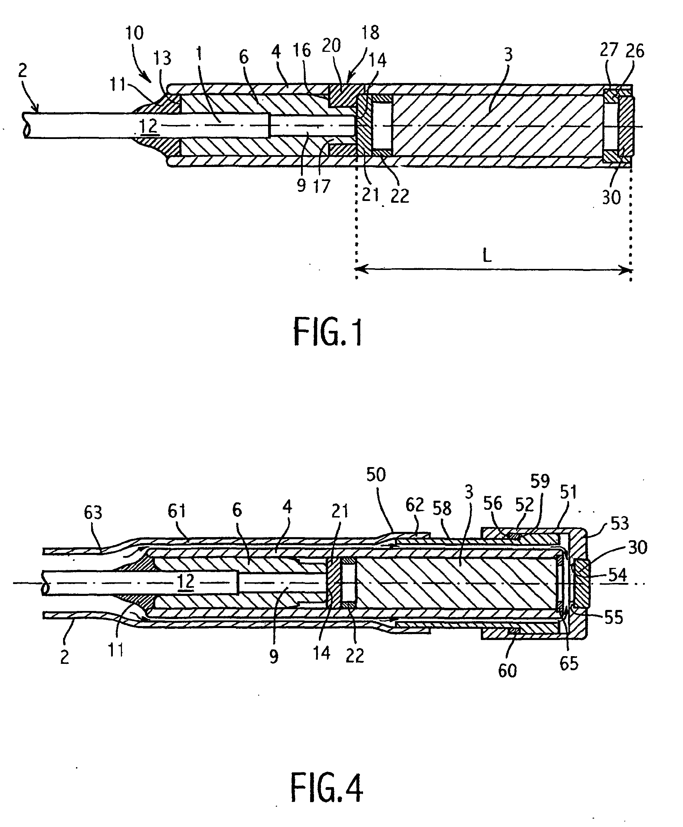

Selon l'exemple de réalisation choisi et représenté sur la figure 1, la tête optique se compose d'une structure mécanique au sein de laquelle d'un côté est introduite et fixée la partie terminale distale 1 d'un faisceau ordonné 2 de fibres optiques souples et de l'autre côté sont logés des moyens optiques permettant la focalisation d'un signal sortant d'une ou plusieurs fibres dudit faisceau de fibres.

La structure mécanique comporte un tube porte-optique 4 de section circulaire. Le faisceau 2 est constitué de fibres optiques souples qui sont ordonnées de la même manière en entrée et en sortie du faisceau, et entourées d'une gaine 12. Un embout métallique tubulaire 6, ouvert de part et d'autre est enfilé et ajusté sur la partie terminale 1 du faisceau 2 de sorte que l'extrémité 14 du faisceau 2 affleure à l'extrémité de l'embout 6. L'embout 6 permet préalablement à l'assemblage dans le tube porte-optique 4 le polissage de l'extrémité 14 du faisceau de fibres. A cet effet, la partie terminale 1 du faisceau de fibres 2 comporte une portion dénudée 9. Grâce à un état de surface le plus parfait possible de l'extrémité 14, on minimise les réflexions parasites à l'entrée et la sortie des fibres et on accroît la qualité du signal. L'embout 6 est inséré de manière ajustée dans le tube porte-optique 4. Du côté de l'extrémité arrière 10 de la tête optique, le faisceau de fibres 2 est fixé à l'aide d'un point de colle adéquate 11 (biocompatible et assurant l'étanchéité) reliant la gaine 12 du faisceau de fibres 2, la face arrière 13 de l'embout 6 et le tube porte-optique 4, l'embout 6 se trouvant légèrement en retrait dans le tube porte-optique. Du côté de l'extrémité dénudée 9 du faisceau de fibres 2, l'embout 6 présente un épaulement annulaire 16 en retrait par rapport à la surface extérieure de l'embout définissant une extrémité 17 de diamètre rétréci. Une ouverture 18 est présente dans le tube porte-optique 4 destinée à venir en regard de l'extrémité rétrécie 17 de l'embout 6 de manière à pouvoir ajuster la position de l'embout 6 et introduire un deuxième point de colle adéquate 20. Cela permet également de coller à sa périphérie une lame 21 d'adaptation d'indice, à faces planes et parallèles, ladite lame étant placée au contact de l'extrémité 14 du faisceau de fibres 2 et de l'extrémité 17 de l'embout 6. Le diamètre de la lame 21 correspond au diamètre intérieur du tube porte-optique 4. Les caractéristiques de la lame 21, nature et épaisseur, sont choisies pour obtenir un bon compromis entre le taux de rétrodiffusion et une résistance suffisante pour l'intégration mécanique. Son indice est choisi pour être très proche de celui du coeur des fibres. La lame 21 grâce à cet indice et le choix de son épaisseur permet de minimiser et de rejeter hors du plan focal la réflexion qui s'opère à l'extrémité distale du guide d'image en réalisant une adaptation d'indice. En contact de la périphérie de la lame 21 est prévue une entretoise tubulaire 22 servant à espacer d'une longueur donnée un bloc optique de focalisation 3 (qui sera décrit plus loin en détails), suivi au contact d'une seconde entretoise 26 tubulaire servant à espacer un hublot de sortie 30. Dans cette partie d'extrémité avant 19 de la tête optique, le tube porte-optique 4 présente un épaulement annulaire interne 27, en retrait contre lequel est adapté à prendre appui la face arrière de l'entretoise 26. De même un épaulement annulaire 28 est prévu dans la face interne de l'entretoise 26 contre lequel est positionné la périphérie de la face arrière du hublot de sortie 30. L'extrémité de l'entretoise 26 et le hublot 30 affleurent à l'extrémité 19 de la tête optique. Le hublot de sortie 30 est une lame à faces parallèles et planes, présentant ici aussi une épaisseur suffisante pour assurer une bonne résistance lors de l'insertitin mécanique. Il est collé à sa périphérie au contact de l'entretoise 26. Lorsqu'il est destiné à venir en contact d'un tissu, le hublot est choisi de manière à être chimiquement neutre. Il permet à la fois de réaliser une adaptation d'indice par rapport au site d'observation de la même façon qu'à la sortie du faisceau de fibres 2, ce qui engendre une minimisation de la réflexion s'opérant sur la surface d'analyse. Dans le cas de l'observation d'un tissu biologique, un traitement anti-reflet dans l'eau pourra en outre être réalisé afin de mieux s'adapter à l'indice des tissus, et ainsi d'améliorer le contraste de l'image. Le système optique est selon l'invention télécentrique dans l'espace image.

L'assemblage de la tête optique se fait de la manière suivante : l'embout 6 est enfilé sur la partie terminale du faisceau de fibres optiques ayant une portion d'extémité dénudée ; l'ensemble est ensuite inséré et ajusté dans le tube porte-optique 4 en faisant coïncider l'ouverture 18 dudit tube 4 avec la portion rétrécie 17 de l'embout 6 ; par l'autre extrémité du tube porte-optique 4 est enfilée la lame 21 pour qu'elle vienne au contact de l'extrémité 14 du faisceau de fibres ; puis on enfile l'entretoise 22, le bloc optique 3, l'entretoise 26 et pour finir le hublot 30 ; on met les points de colle 11 et 20 pour fixer l'embout 6 et la lame 21.

Le bloc optique 3 comprend un ensemble de lentilles ayant pour fonction de focaliser un faisceau d'excitation en un point focal d'excitation situé dans un plan d'analyse subsurfacique XY perpendiculaire à l'axe optique. Le choix de la position (dans un plan extra-focal), de la courbure et d'un traitement anti-reflet optimal permet d'éviter que le signal réfléchi par les lentilles ne viennent parasiter le signal provenant de l'échantillon (le couplage du signal réfléchi ne doit pas dépasser 10-5 par rapport au signal en sortie de fibre).

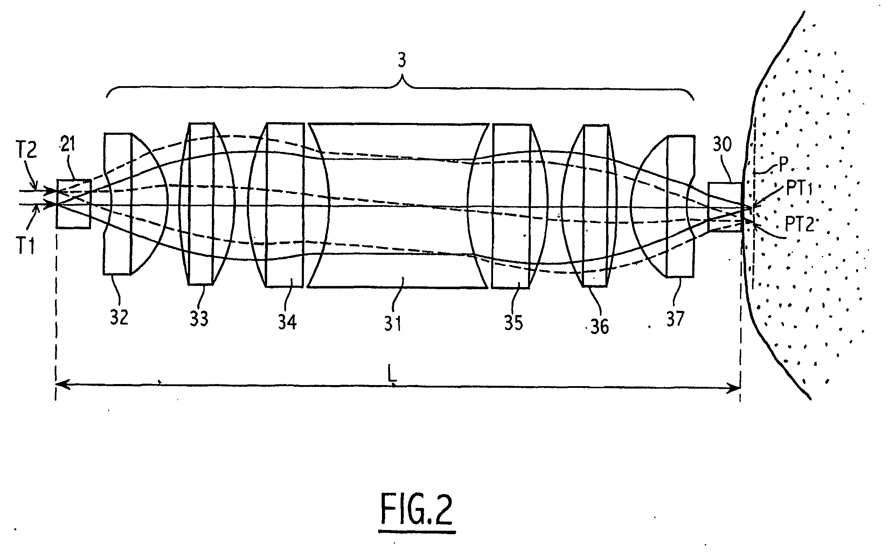

A titre d'exemple, on a représenté sur la figure 2 un bloc optique 3 à grandissement unitaire comprenant symétriquement de part et d'autre d'une lentille bi-concave 31 en verre BK7, en sortie de la lame 21: un ménisque 32 en verre SF6, une lentille bi-convexe 33 en verre BK7 et une lentille plan-convexe 34 en verre SF6, et en amont du hublot de sortie 30 une lentille plan-convexe 35 en verre SF6, une lentille bi-convexe 36 en verre BK7 et un ménisque 37 en verre SF6.

La figure 2 montre schématiquement le trajet optique d'un faisceau d'excitation émergeant du faisceau de fibres optiques. On a représenté un premier trajet optique T1 en trait plein d'un faisceau principal centré sur l'axe optique du système et en pointillés un second trajet optique T2 d'un faisceau émergent d'une fibre optique ou d'un groupe de fibres ne se trouvant pas sur l'axe optique. Le faisceau émergent du hublot 30 converge en un point focal d'excitation, par exemple PT1 ou PT2, situé dans un plan d'analyse XY subsurfacique. Le signal rétroémis par le point focal d'excitation empreinte ensuite en sens inverse le même chemin optique.

Les caractéristiques détaillées (courbure, position etc.) des différentes lentilles 31 à 37 selon un mode de réalisation particulier ainsi que de la lame 21 et du hublot de sortie 30 sont données dans le tableau 1 ci-après.

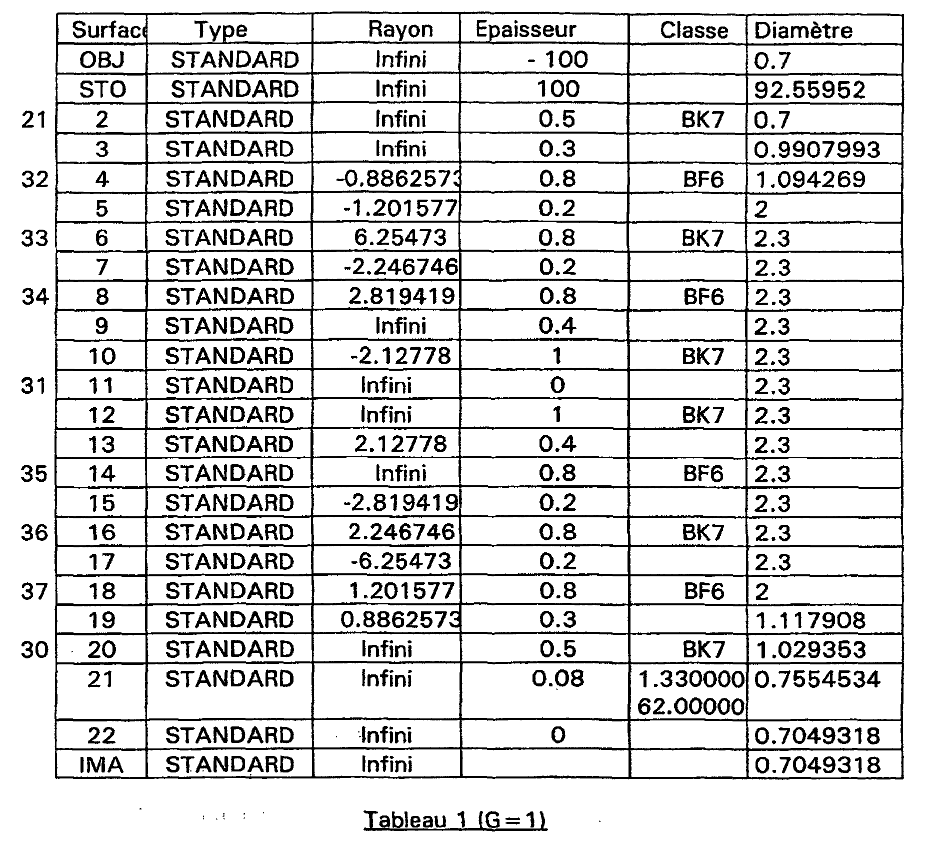

La construction selon l'invention est miniaturisable tout en autorisant une qualité de signal de très bonne qualité, comme le montre les caractéristiques ci-après, données à titre d'exemple, pour une tête optique présentant les caractéristiques, qui viennent d'être décrites en référence à la figure 1 et destinée à être insérée dans le canal opérateur d'un endoscope et mettant en oeuvre à l'extrémité proximale du guide de signal des moyens d'imagerie confocale comprenant : une source de lumière (par exemple un laser pulsé), des moyens de balayage pour injecter le faisceau produit fibre à fibre de manière adressée, des moyens de filtrage temporel et spatial du signal rétrodiffusé, des moyens de détection, des moyens de traitement du signal et des moyens de présentation d'image, tels que décrits notamment dans la demande internationale WO 00/16151.
Caractéristiques d'une tête optique selon l'invention pour un coloscope ou gastroscope :
Dimensions :
   - 2,5 mm de diamètre extérieur pour le tube porte-optique ;
   - un faisceau de fibres 2 par exemple de marque Sumitomo® constitué de 30 000 fibres de diamètre de coeur de 2,5 µm et d'espacement inter-coeur de 4 µm ou de marque Fujikura® constitué de 30 000 fibres de diamètre de coeur de 1,9µm et d'espacement inter-coeur de 3,3 µm;
   - un bloc optique 3 de 1,8 mm de diamètre;
   - une longueur L (voir figure 1) entre la sortie de la fibre du guide de signal et la face externe du hublot de sortie 30 de 8,75 mm, avec une frontale variant de 50 à 150 µm ;
   - une longueur totale comprenant L et la reprise mécanique rigide sur faisceau de fibres optiques de 16,6 mm, compatible avec le rayon de courbure du canal opérateur d'un coloscope courant (Rc=40mm) ;
   - 0,5 mm d'épaisseur pour la lame d'adaptation d'indice 21 et pour le hublot de sortie 30, suffisante lors de l'insertion mécanique et permettant un taux de rétrodiffusion de l'ordre de 3.10⁻⁴.
Température de fonctionnement : 37°C.
   Qualité de l'image
   - qualité d'image proche de la limite de diffraction ; l'erreur du front d'onde (WFE, "wave front error" en anglais) dans tout le champ est de l'ordre de λ/30 au centre du champ à λ/20 en bord de champ ; cette excellente qualité d'image assure un bon couplage retour dans la fibre d'excitation (~90%);
   - FTM (fonction de transfert de modulation) : elle correspond au tracé de l'intensité relative en fonction de la fréquence spatiale. La fréquence de coupure est définie par 1/(2d) où d correspond à la distance inter-coeur des fibres, et est exprimée en cycles/mm. Ici, avec une distance inter-coeur de 4pm, la fréquence de coupure est de 125 cycles/mm. La FTM permet d'évaluer la qualité de l'image en comparant la courbe à celle de la limite de diffraction, et en utilisant le critère selon lequel le contraste doit être de 0,5 (valeur de l'intensité relative donnée par la courbe) à la fréquence spatiale maximale du dispositif, en l'occurrence 125 cycles/mm dans le cas présent. Le résultat obtenu ici est effectivement proche de la limite de diffraction, présentant un contraste de 0,75 à la fréquence spatiale de 125 cycles/mm, donc assure une très bonne qualité d'image ;
   - Energie encerclée : elle permet d'évaluer la résolution latérale que l'on est en mesure d'attendre, en évaluant le pourcentage d'énergie contenue dans un diamètre. Afin de résoudre une tâche de diamètre φ, il faut que 50% de l'énergie minimum soit contenue dans ce diamètre. Dans le cas présent, 50% de l'énergie provenant du point objet est encerclée dans un diamètre de 1,5*µ*m, quelle que soit sa position dans le champ. 50% de l'énergie provenant d'une fibre optique du guide de signal (de diamètre de coeur de 2,5 µm) est donc encerclée dans un diamètre de 4 µm.
   - Courbure de champ, distorsion : L'image est courbée de 31 µm à 35 µm entre le centre et le bord du champ. La courbure de champ résiduelle est très faible (de l'ordre de 2 µm), ainsi que la distorsion (de l'ordre de 0,8%).
Transmission
   - sur un trajet : de l'ordre de 0,97%.

Ainsi, la solution proposée selon l'invention est effectivement miniaturisable et permet d'obtenir une image de très bonne qualité présentant une résolution latérale attendue (à savoir 4 µm) et d'optimiser le rapport signal à bruit en minimisant la réflexion parasite en sortie de guide d'image, en optimisant le taux de couplage en retour et la transmission du système. Cette solution répond au problème posé et offre les avantages de la simplicité de montage et du faible coût.

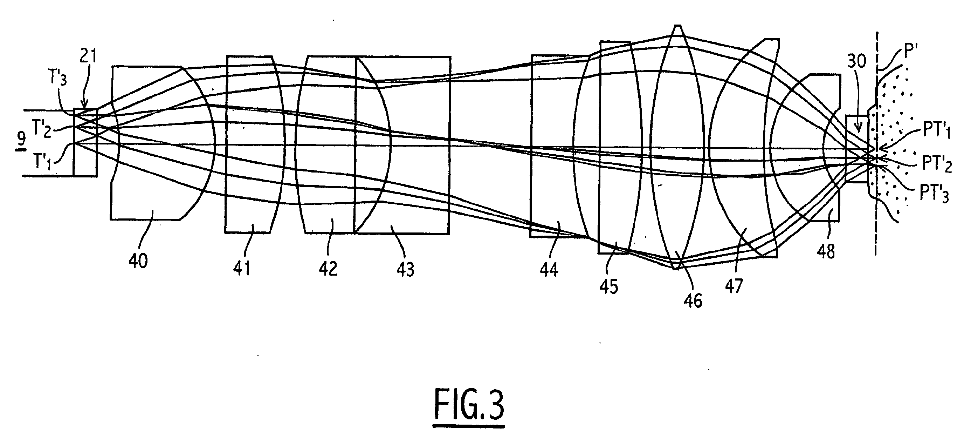

Il va de soi que des variantes de réalisation de l'invention sont possibles notamment à la figure 3 est représenté un bloc optique 3 de focalisation à grandissement 0,5 (les mêmes références sont utilisées pour les éléments communs à la figure 1). En sortie de la lame d'adaptation d'indice 21 sont agencés successivement un ménisque 40 en verre SF6, un plan-convexe 41 en verre BK7, un plan-convexe 42 en SF6, un plan-concave 43 en BK7, un plan-concave 44 en BK7, un plan-convexe 45 en SF6, une lentille bi-convexe 46 en BK7, un ménisque 47 en SF6 et un ménisque 48 en SF6. De même qu'à la figure 2, on a représenté ici trois trajets optiques émanant d'une fibre différente du faisceau : T'₁, centré sur l'axe optique, formant un point de focalisation PT'₁ dans un plan subsurfacique P', et T'₂ et T'₃, des rayons marginaux non centrés formant respectivement un point de focalisation PT'₂ et PT'₃ dans le plan P'.

Les caractéristiques détaillées selon un mode particulier de réalisation (courbure, position etc.) des différentes lentilles 40 à 48 ainsi que de la lame 21 et du hublot de sortie 30 sont données dans le tableau 2 ci-après.

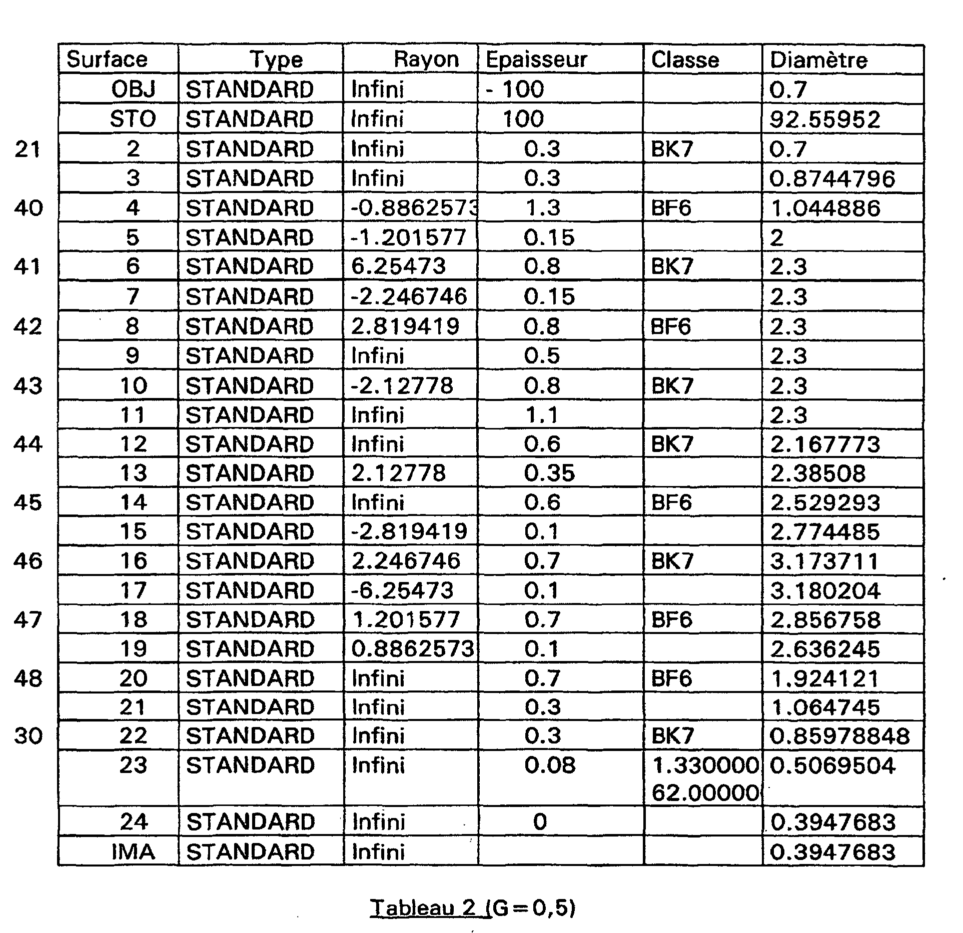

Le grandissement non unitaire, en l'occurrence de 0,5 depuis l'extrémité distale du guide d'image jusqu'au plan d'analyse dans cet exemple d'application, permet d'obtenir :
- une meilleure résolution latérale (PSF de 0,75 *µ*m pour un objet étendu de diamètre égal au diamètre de coeur d'une fibre (1,9µm), contre 1,4µm pour une tête optique à grandissement unitaire).
- Une meilleure résolution axiale (de l'ordre de 5 *µ*m contre 10 *µ*m pour la tête optique à grandissement unitaire)
- Une ouverture numérique d'illumination plus grande (de l'ordre de 0,9 contre 0,42 pour la tête optique à grandissement unitaire), et par conséquent une image plus contrastée.

Sur la figure 4 est représentée une autre forme de réalisation de tête optique selon l'invention comportant des moyens de type hydrauliques pour faire varier la profondeur du plan d'analyse P. Les éléments similaires à ceux de la figure 1 portent les mêmes références. La tête se différencie de celle de la figure 1 par le fait que le hublot 30 est porté par un capuchon, portant la référence globale 50, qui est enfilé sur la tête optique. Ce capuchon comprend une partie d'extrémité 51 avec une jupe 52 et une paroi avant 53 dans laquelle est ménagée une ouverture 54 avec un rebord annulaire 55 adaptés à recevoir le hublot 30, la périphérie de ce dernier étant collée sur le rebord 55 avec une colle appropriée. Le diamètre extérieur de cette partie d'extrémité 51 peut être de 3 mm environ, dimension compatible avec le canal opérateur d'un endoscope. La jupe 52 est enfilée sur une partie tubulaire dite intermédiaire 58 du capuchon 50, des moyens d'accouplement étant prévus entre ces deux parties comprenant sur la face interne de la jupe 52 en retrait un rebord annulaire 56 et sur la face externe de la partie intermédiaire 58 un épaulement 59, un joint compressible annulaire 60 étant ménagé entre lesdites parties, assurant l'étanchéité du couplage. Enfin, le capuchon 50 comporte une partie arrière 61, destinée au raccordement avec une arrivée d'air, dont le diamètre de l'extrémité avant 62 est élargi pour s'enfiler sur l'extrémité arrière de la partie intermédiaire 58 et le diamètre arrière 63 est rétréci pour s'adapter au diamètre du faisceau de fibres optiques 2. Le capuchon 50 présente globalement un diamètre intérieur plus grand que le diamètre externe du tube porte-optique, de sorte qu'un espace est ménagé entre le capuchon 50 et la tête optique qu'il est prévu de mettre en communication avec l'arrivée d'air. Ainsi, selon l'invention, le réglage de la position du plan focal subsurfacique se fait non pas en modifiant la position des lentilles à l'intérieur du bloc optique 3 mais en modifiant la position du hublot 30 par rapport audit bloc optique 3, grâce à un capuchon mobile 50 actionné pneumatiquement portant ledit hublot.

La tête qui vient d'être décrite se différencie également de celle décrite en référence à la figure 1, par le fait qu'il n'est pas prévu de point de colle tel que 20 sur la figure 1 pour fixer l'extrémité de l'embout 6. La fixation se fait ici grâce au point de colle 11 à l'arrière de la tête et d'un anneau 65 fixé à l'extrémité du tube porte-optique 4 contre un épaulement.

## Revendications

1. Tête optique miniaturisée prévue pour équiper l'extrémité distale d'un faisceau de fibres optiques souples (2), ladite tête optique étant destinée à venir au contact d'une surface d'analyse et comprenant des moyens optiques (3) pour focaliser un signal d'excitation sortant dudit faisceau de fibres en un point focal dit d'excitation situé à une profondeur donnée sous la surface d'analyse et pour prélever un signal rétroémis par le point focal d'excitation qui est véhiculé en retour par ledit faisceau de fibres, **caractérisée par** un tube porte-optique (4) de section circulaire au sein duquel sont introduits d'un côté la partie terminale distale (1) du faisceau de fibres (2) et de l'autre les moyens optiques, ces derniers comprenant une lame (21) placée au contact de l'extrémité (14) du faisceau de fibres dont l'indice est proche de celui du coeur des fibres et un bloc optique de focalisation (3), un hublot de sortie (30) étant en outre destiné à venir au contact de la surface d'analyse et adapté à réaliser une adaptation d'indice de manière à s'affranchir de la réflexion parasite s'opérant sur la surface d'analyse.

2. Tête optique selon la revendication 1, **caractérisée en ce que** le bloc optique comporte un ensemble de lentilles, chaque lentille étant positionnée dans un plan extra-focal permettant d'éviter que le signal réfléchi par les lentilles ne viennent parasiter le signal provenant de l'échantillon.

3. Tête optique selon la revendication 1 ou 2, **caractérisée en ce que** le bloc optique comporte un ensemble de lentilles, chaque lentille présentant un traitement anti-reflet optimal permettant d'éviter que le signal réfléchi par les lentilles ne viennent parasiter le signal provenant de l'échantillon.

4. Tête optique selon l'une des revendications précédentes, **caractérisée en ce que** le bloc optique comporte un ensemble de lentilles, chaque lentille présentant une courbure adaptée afin d'éviter que le signal réfléchi par les lentilles ne viennent parasiter le signal provenant de l'échantillon.

5. Tête optique selon l'une quelconque des revendications précédentes, **caractérisée en ce que** le hublot (30) est inséré à l'extrémité du tube porte-optique (4), une entretoise tubulaire (26) étant ménagée entre le bloc optique (3) et ledit hublot (30).

6. Tête optique selon la revendication 5, **caractérisée en ce que** la périphérie du hublot (30) est placée en butée contre un épaulement ménagé en retrait à l'intérieur de l'entretoise (26).

7. Tête optique selon la revendication 5 ou 6, **caractérisée en ce que** l'entretoise tubulaire (26) est placée en butée contre un épaulement (27) ménagé en retrait à l'intérieur du tube porte-optique.

8. Tête optique selon l'une quelconque des revendications 1 à 4, **caractérisée en ce que** le hublot (30) est porté par un capuchon mobile (50) enfilé sur l'extrémité de la tête optique, des moyens étant prévus pour déplacer ledit capuchon de manière à faire varier le plan focal de visualisation d'une profondeur donnée.

9. Tête optique selon la revendication 8, **caractérisée en ce que** le capuchon mobile (50) comprend une partie d'extrémité (51) avec une face avant (53) dans laquelle est ménagée une ouverture pour le hublot (30), ladite partie d'extrémité (51) étant accouplée à une partie intermédiaire (58) du capuchon mobile (50) et un joint d'étanchéité compressible (60) étant ménagé au niveau de l'accouplement.

10. Tête optique selon la revendication 9, **caractérisée en ce que** le hublot (30) est collé contre un épaulement (55) ménagé à cet effet dans l'ouverture de la partie d'extrémité (51).

11. Tête optique selon l'une des revendications 8 à 10, **caractérisée en ce qu'**un espace est ménagé entre le capuchon mobile (50) et la tête optique de en communication avec une arrivée d'air, le réglage de l'arrivée d'air permettant de déplacer ledit capuchon et donc la position du hublot de sortie (30).

12. Tête optique selon l'une des revendications 1 à 7, **caractérisée par** un moyen optique mobile prévu dans le bloc optique adapté à être déplacé axialement.

13. Tête optique selon la revendication 12 **caractérisée en ce que** le déplacement est réalisé à l'aide d'un moteur piézo-électrique.

14. Tête optique selon l'une des revendication 1 à 7 **caractérisée par** un moyen optique dans le bloc optique possédant un rayon de courbure modifiable de manière à changer sa distance focale et donc la profondeur du plan d'observation.

15. Tête optique selon la revendication 14, **caractérisée en ce que** le moyen optique est un moyen optique liquide.

16. Tête optique selon l'une des revendications précédentes, **caractérisée par** la présence d'un embout tubulaire (6) ménagé autour de la partie terminale (1) du faisceau de fibres (2), l'extrémité (14) du faisceau de fibres affleurant à l'extrémité dudit embout.

17. Tête optique selon la revendication 16, **caractérisée en ce que** le faisceau de fibres optiques (2), comportant une gaine (12), dans sa portion entourée de l'embout (6) présente une portion terminale dénudée (9).

18. Tête optique selon la revendication 16 ou 17, **caractérisée en ce que** l'embout (6) se trouve sensiblement en retrait à l'intérieur du tube porte-optique (4) de sorte qu'un point de colle (11) puisse être ménagé au contact de l'extrémité arrière (13) de l'embout (6), de l'extrémité arrière du tube porte-optique (4) et de la gaine (12) du faisceau de fibres (2).

19. Tête optique selon l'une des revendications 16 à 18, **caractérisée en ce que** l'embout (6) du côté de la lame d'adaptation d'indice (21) comporte une extrémité de diamètre rétrécie (17) en regard de laquelle est ménagé une ouverture (18) dans le tube porte-optique (4) et ladite lame (21) présentant un diamètre extérieur correspondant au diamètre intérieur du tube porte-optique (4) de sorte qu'un point de colle (20) puisse être ménagé au contact de ladite extrémité d'embout (17), de la périphérie de ladite lame (21) et du tube porte-optique (4).

20. Tête optique selon l'une quelconque des revendications précédentes, **caractérisée par** une entretoise tubulaire (22) ménagée entre la lame d'adaptation d'indice (21) et le bloc optique de focalisation (3).

21. Application de la tête optique selon l'une quelconque des précédentes à un endoscope à imagerie confocale.

22. Application de la tête optique selon la revendication 21 à un endoscope à imagerie confocale utilisant un faisceau de fibres optiques balayées une à une à l'extrémité proximale dudit faisceau.

## Patentansprüche

1. Miniaturisierter optischer Kopf, der zum Bestücken des distalen Endes eines Bündels von flexiblen optischen Fasern (2) vorgesehen ist und dazu bestimmt ist, mit einer Analysefläche in Kontakt zu kommen, und optische Mittel (3) zum Fokussieren eines aus dem Faserbündel austretenden Erregungssignals an einem sogenannten Erregungs-Brennpunkt umfasst, der in einer gegebenen Tiefe unter der Analysefläche gelegen ist, und zum Entnehmen eines von dem Erregungs-Brennpunkt rückübertragenen Signals, das von dem Faserbündel zurückbefördert wird, **gekennzeichnet durch** ein Optikträgerrohr (4) mit kreisförmigem Querschnitt, in das auf einer Seite der distale Endteil (1) des Faserbündels (2) und auf der anderen Seite die optischen Mittel eingeführt sind, wobei diese eine im Kontakt mit dem Ende (14) des Faserbündels angeordnete Lamelle (21) aufweist, deren Index nahe dem des Kerns der Fasern ist, und einen optischen Fokussierungsblock (3) aufweist, wobei ein Austrittsfenster (30) ferner dazu bestimmt ist, mit der Analysefläche in Kontakt zu kommen, und dafür ausgelegt ist, eine Indexadaption vorzunehmen, um die auf der Analysefläche auftretende Störreflexion zu vermeiden.

2. Optischer Kopf nach Anspruch 1, **dadurch gekennzeichnet, dass** der optische Block eine Linseneinheit umfasst, wobei jede Linse in einer extrafokalen Ebene angeordnet ist, wodurch vermieden werden kann, dass das von den Linsen reflektierte Signal das von der Probe kommende Signal stört.

3. Optischer Kopf nach Anspruch 1 oder 2, **dadurch gekennzeichnet, dass** der optische Block eine Linseneinheit umfasst, wobei jede Linse eine optimale Antireflexionsbehandlung aufweist, durch die vermieden werden kann, dass das von den Linsen reflektierte Signal das von der Probe kommende Signal stört.

4. Optischer Kopf nach einem der vorhergehenden Ansprüche, **dadurch gekennzeichnet, dass** der optische Block eine Linseneinheit aufweist, wobei jede Linse eine angepasste Krümmung besitzt, um zu vermeiden, dass das von den Linsen reflektierte Signal das von der Probe kommende Signal stört.

5. Optischer Kopf nach einem der vorhergehenden Ansprüche, **dadurch gekennzeichnet, dass** das Fenster (30) am Ende des Optikträgerrohrs (4) eingesetzt ist, wobei zwischen dem optischen Block (3) und dem Fenster (30) ein Abstandsrohr (26) vorgesehen ist.

6. Optischer Kopf nach Anspruch 5, **dadurch gekennzeichnet, dass** der Umfang des Fensters (30) in Anschlag an einer Schulter angeordnet ist, die im Inneren des Abstandsrohrs (26) zurückversetzt angeordnet ist.

7. Optischer Kopf nach Anspruch 5 oder 6, **dadurch gekennzeichnet, dass** das Distanzrohr (26) in Anschlag an einer Schulter (27) angeordnet ist, die im Inneren des Optikträgerrohrs zurückversetzt vorgesehen ist.

8. Optischer Kopf nach einem der Ansprüche 1 bis 4, **dadurch gekennzeichnet, dass** das Fenster (30) von einer auf das Ende des optischen Kopfs aufgesetzten, abnehmbaren Kappe (50) getragen ist, wobei Mittel vorgesehen sind, um die Kappe so zu bewegen, dass die Abbildungsbrennweite einer gegebenen Tiefe geändert wird.

9. Optischer Kopf nach Anspruch 8, **dadurch gekennzeichnet, dass** die bewegliche Kappe (50) einen Endteil (51) mit einer Vorderseite (53) aufweist, in der eine Öffnung für das Fenster (30) vorgesehen ist, wobei dieser Endteil (51) mit einem Zwischenteil (58) der beweglichen Kappe (50) gekuppelt ist und ein komprimierbarer Dichtring (60) auf Höhe der Kupplung vorgesehen ist.

10. Optischer Kopf nach Anspruch 9, **dadurch gekennzeichnet, dass** das Fenster (30) an einer zu diesem Zweck in der Öffnung des Endteils (51) vorgesehenen Schulter (55) angeklebt ist.

11. Optischer Kopf nach einem der Ansprüche 8 bis 10, **dadurch gekennzeichnet, dass** zwischen der beweglichen Kappe (50) und dem optischen Kopf vorgesehen ist, der mit einer Luftzufuhr in Verbindung ist, wobei die Regelung der Luftzufuhr die Bewegung der Kappe und damit der Stellung des Austrittsfensters (30) gestattet.

12. Optischer Kopf nach einem der Ansprüche 1 bis 7, **gekennzeichnet durch** ein in dem optischen Block vorgesehenes bewegliches optisches Mittel, das dafür ausgelegt ist, axial bewegt zu werden.

13. Optischer Kopf nach Anspruch 12, **dadurch gekennzeichnet, dass** die Bewegung mit Hilfe eines piezoelektrischen Motors ausgeführt wird.

14. Optischer Kopf nach einem der Ansprüche 1 bis 7, **gekennzeichnet durch** ein optisches Mittel in dem optischen Block, das eine veränderlichen Krümmungsradius besitzt, so dass die Brennweite und damit die Tiefe der Beobachtungsebene geändert wird.

15. Optischer Kopf nach Anspruch 14, **dadurch gekennzeichnet, dass** das optische Mittel ein flüssiges optisches Mittel ist.

16. Optischer Kopf nach einem der vorhergehenden Ansprüche, **gekennzeichnet durch** das Vorhandensein eines rohrförmigen Ansatzes (6), der um den Endteil (1) des Faserbündels (2) herum vorgesehen ist, wobei das Ende (14) des Faserbündels mit dem Ende dieses Ansatzes bündig ist.

17. Optischer Kopf nach Anspruch 16, **dadurch gekennzeichnet, dass** das optische Faserbündel (2), das eine Hülle (12) aufweist, in seinem von dem Ansatz (6) umgebenen Abschnitt einen freigelegten Endabschnitt (9) aufweist.

18. Optischer Kopf nach Anspruch 16 oder 17, **dadurch gekennzeichnet, dass** der Ansatz (6) im Inneren des Optikträgerrohrs (4) im wesentlichen zurückversetzt ist, so dass eine Klebstoffpunkt (11) im Kontakt mit dem hinteren Ende (13) des Ansatzes (6), dem hinteren Ende des Optikträgerrohrs (4) und der Hülle (12) des Faserbündels (2) vorgesehen werden kann.

19. Optischer Kopf nach einem der Ansprüche 16 bis 18, **dadurch gekennzeichnet, dass** der Ansatz (6) auf der Seite der Indexanpassungslamelle (21) ein Ende (17) mit verringertem Durchmesser aufweist, welchem gegenüber eine Öffnung (18) in dem Optikträgerrohr (4) vorgesehen ist, wobei die Lamelle (21) einen Außendurchmesser aufweist, der dem Innendurchmesser des Optikträgerrohrs (4) entspricht, so dass ein Klebstoffpunkt (20) im Kontakt mit diesem Ansatzende (17), dem Umfang der Lamelle (21) und dem Optikträgerrohr (4) vorgesehen werden kann.

20. Optischer Kopf nach einem der vorhergehenden Ansprüche, **gekennzeichnet durch** ein Abstandsrohr (22), das zwischen die Indexanpassungslamelle (21) und den optischen Fokussierungsblock (3) vorgesehen ist.

21. Anwendung des optischen Kopfs nach einem der vorhergehenden Ansprüche auf ein Endoskop mit konfokaler Abbildung.

22. Anwendung des optischen Kopfs nach Anspruch 21 auf ein Endoskop mit konfokaler Abbildung, das ein Bündel von optischen Fasern verwendet, die am proximalen Ende des Bündels nacheinander bestrichen werden.

## Claims

1. Miniaturized optical head provided for equipping the distal end of a flexible optical fibre bundle (2), said optical head being intended to come into contact with an analyzing surface and comprising optical means (3) for focussing an excitation signal coming out from said fibre bundle into a so-called excitation focal point situated at a given depth beneath the analyzing surface and for sampling a signal backscattered by the excitation focal point which is carried back by said fibre bundle, **characterized by** an optics-holder tube (4) circular in section wherein are inserted on one side the distal end portion (1) of the fibre bundle (2) and on the other the optical means, the latter comprising a plate (21) placed in contact with the end (14) of the fibre bundle the index of which is close to that of the fibre core and a focussing optical block (3), an output window (30) being moreover intended to come into contact with the analyzing surface and adapted to produce an index adaptation in order to eliminate parasitic reflection occurring on the analyzing surface.

2. Optical head according to claim 1, **characterized in that** the optical block comprises a set of lenses, each lens being positioned in an extra-focal plane making it possible to avoid the signal reflected by the lenses causing interference to the signal originating from the sample.

3. Optical head according to claim 1 or 2, **characterized in that** the optical block comprises a set of lenses, each lens having an optimal anti-reflection treatment making it possible to avoid the signal reflected by the lenses causing interference to the signal originating from the sample.

4. Optical head according to one of the preceding claims, **characterized in that** the optical block comprises a set of lenses, each lens have a curvature adapted in order to avoid the signal reflected by the lenses causing interference to the signal originating from the sample.

5. Optical head according to any one of the preceding claims, **characterized in that** the window (30) is inserted at the end of the optics-holder tube (4), a tubular spacer (26) being arranged between the optical block (3) and said window (30).

6. Optical head according to claim 5, **characterized in that** the periphery of the window (30) is placed abutting against a collar arranged in recess inside the spacer (26).

7. Optical head according to claim 5 or 6, **characterized in that** the tubular spacer (26) is placed abutting against a collar (27) arranged in recess inside the optics-holder tube.

8. Optical head according to any one of claims 1 to 4, **characterized in that** the window (30) is carried by a mobile cap (50) coupled onto the end of the optical head, means being provided for displacing said cap in such a manner as to vary the focal plane of visualization of a given depth.

9. Optical head according to claim 8, **characterized in that** the mobile cap (50) comprises an end portion (51) with a front surface (53) in which is arranged an opening for the window (30), said end portion (51) being coupled to an intermediate portion (58) of the mobile cap (50) and a compressible seat (60) being arranged at the level of the coupling.

10. Optical head according to claim 9, **characterized in that** the window (30) is glued against a collar (55) arranged for this purpose in the opening of the end portion (51).

11. Optical head according to one of claims 8 to 10, **characterized in that** a space is arranged between the mobile cap (50) and the optical head in communication with an air supply, the adjustment of the air supply allowing displacement of said cap and therefore of the position of the output window (30).

12. Optical head according to one of the claims 1 to 7, **characterized by** a mobile optical means provided in the optical block adapted to be displaced axially.

13. Optical head according to claim 12 **characterized in that** the displacement is carried out using a piezo-electric motor.

14. Optical head according to one of claims 1 to 7 **characterized by** an optical means in the optical block possessing a radius of curvature which can be modified in order to change its focal distance and therefore the depth of the observation plane.

15. Optical head according to claim 14, **characterized in that** the optical means is a liquid optical means.

16. Optical head according to one of the preceding claims, **characterized by** the presence of a tubular joining piece (6) arranged about the end portion (1) of the fibre bundle (2), the end (14) of the fibre bundle being flush with the end of said joining piece.

17. Optical head according to claim 16, **characterized in that** the optical fibre bundle (2), comprising a sheath (12), in its portion surrounded by the joining piece (6) has a bare end portion (9).

18. Optical head according to claim 16 or 17, **characterized in that** the joining piece (6) is situated somewhat retracted inside the optics-holder tube (4) in such a manner that a spot of glue (11) can be arranged in contact with the rear end (13) of the joining piece (6), the rear end of the optics-holder tube (4) and the sheath (12) of the fibre bundle (2).

19. Optical head according to one of claims 16 to 18, **characterized in that** the joining piece (6) on the side of the index adaptation plate (21) comprises an end with a narrow diameter (17) facing which is arranged an opening (18) in the optics-holder tube (4) and said plate (21) having an external diameter corresponding to the internal diameter of the optics-holder tube (4) in such a manner that a spot of glue (20) can be arranged in contact with said end of joining piece (17), the periphery of said plate (21) and the optics-holder tube (4).

20. Optical head according to any one of the preceding claims, **characterized by** a tubular spacer (22) arranged between the index adaptation plate (21) and the focussing optical block (3).

21. Use of the optical head according to any one of the preceding claims in a confocal imaging endoscope.

22. Use of the optical head according to claim 21 in a confocal imaging endoscope using a bundle of optical fibres scanned one by one at the proximal end of said bundle.
